(19) Europäisches Patentamt
European Patent Office
Office européen des brevets

(11) Publication number: 0 201 775 A2

# (12) EUROPEAN PATENT APPLICATION

(21) Application number: 86105688.5

(22) Date of filing: 24.04.86

(51) Int. Cl.4: C07D 213/64 , C07C 83/10 , A61K 37/16 , A01N 43/40

(30) Priority: 26.04.85 JP 90459/85

(43) Date of publication of application: 20.11.86 Bulletin 86/47

(84) Designated Contracting States: CH DE FR GB LI

(71) Applicant: AGRO-KANESHO CO., LTD.
No. 4-1, Marunouchi 2-chome
Chiyoda-ku Tokyo(JP)
Applicant: TOYO SODA MANUFACTURING CO., LTD.
No. 4560, Ohaza Tomita Shinnanyou-shi
Yamaguchi-ken(JP)

(72) Inventor: Someya, Shinzo
C-1108, No. 3-20, Nakaarai
Tokorozawa-shi Saitama-ken(JP)
Inventor: Ito, Mikio
No. 484-2, Ohaza Shimokami
Tokuyama-shi Yamaguchi-ken(JP)
Inventor: Nakanishi, Akira
No. 7-1, Shimizu 2-chome
Shinnanyou-shi Yamaguchi-ken(JP)
Inventor: Nonaka, Yuji
No. 230-2, Ohaza Kamimura
Tokuyama-shi Yamaguchi-ken(JP)

(74) Representative: Vossius & Partner
Siebertstrasse 4 P.O. Box 86 07 67
D-8000 München 86(DE)

## (54) Derivative of propionic acid and selective herbicide containing the same.

(57) The derivative of the propionic acid represented by the following general structural formula [1]:

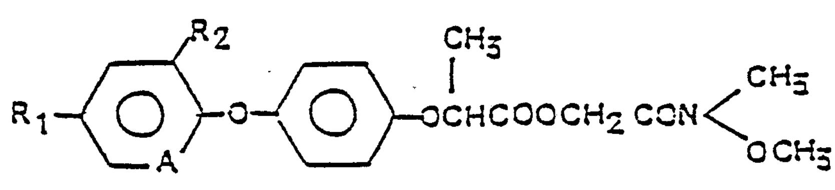

[I]

wherein $R_1$ denotes a chlorine atom, bromine atom, trifluoromethyl group or nitro group; $R_2$ denotes a hydrogen atom or chlorine atom; and A denotes a methine group or nitrogen atom, is quite useful as a selective herbicide.

EP 0 201 775 A2

Derivative of propionic acid and selective herbicide containing the same.

Background of the Invention:

The present invention relates to a novel derivative of phenoxypropionic acid ester, a manufacturing process therefor, a selective herbicide containing said chemical compound as the active constituent and a herbicidal process using said herbicide.

Many derivatives of the phenoxypropionic acid ester have been reported to have herbicidal effect, including, for example, 2-[4-(4-trifluoromethylphenoxy)-phenoxy] propionic acid methyl ester, 2-[4-(2,4-dichlorophenoxy)-phenoxy] propionic acid methyl ester, 2-[4-(3,5-dichloro-2-pyridyloxy)-phenoxy ] propionic acid methyl ester, and 2-[4-(5-trifluoromethyl-2-pyridyloxy)-phenoxy] propionic acid butyl ester. However, the actions of these compounds are not always large enough.

Therefore, there is still a strong demand for a selective herbicide which is capable of killing weeds of Gramineae family without damaging useful broad-leaved crops such as beet, soybean, cotton, alfalfa, rape-seed, potato, sunflower, Japanese radish, Chinese cabbage, cabbage, tomato, etc. regardless of whether it is used in pre-emergence treatment or post-emergence treatment. When herbicides using the conventional compounds of the phenoxy propionic acid ester are used, especially, when they are used after the growth of the weeds, it usually takes more than 8 days before their effects are fully displayed, and farmers growing such vegetables have been eagerly waiting for a solution to such problem.

Therefore, the object of the present invention is to obtain said compound industrially, to provide an improved herbicide meeting the needs of the market, and to provide a herbicidal process using said herbicide.

Summary of the Invention:

The present invention relates to a novel derivative of phenoxypropionic acid ester (hereinafter referred to as the "compound of the present invention") which can be represented by the following general structural formula [1]:

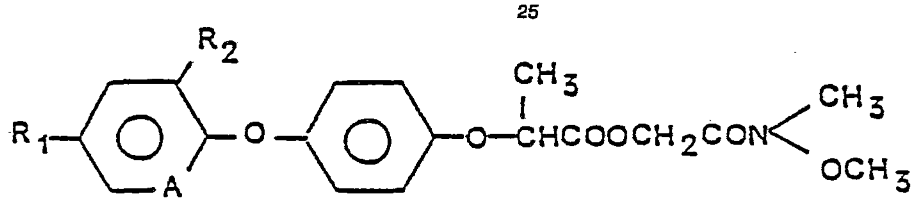

[I]

wherein $R_1$ denotes a chlorine atom, bromine atom, trifluoromethyl group or nitro group; $R_2$ denotes a hydrogen atom or chlorine atom; and A denotes a methine group or nitrogen atom, a manufacturing process of said derivative, a herbicide containing said derivative as the active constituent and a herbicidal process using said herbicide.

Description of the Invention:

The herbicide containing the compound of the present invention is able to kill weeds of Gramineae family such as the barnyard grass, crab grass, goose grass, foxtail, wild oats, water foxtail, yellow foxtail, quack grass, Agropyron tsukushiens, Bermuda grass, Johnson grass, etc. growing among broad-leaved crops such as the beet, sunflower, Japanese radish, Chinese cabbage, cabbage, tomato, etc. without damaging such useful crops, regardless of whether said herbicide is used for a pre-emergence treatment or a post-emergence treatment.

The compound of the present invention can be manufactured by various processes, typical ones of which are as follows:

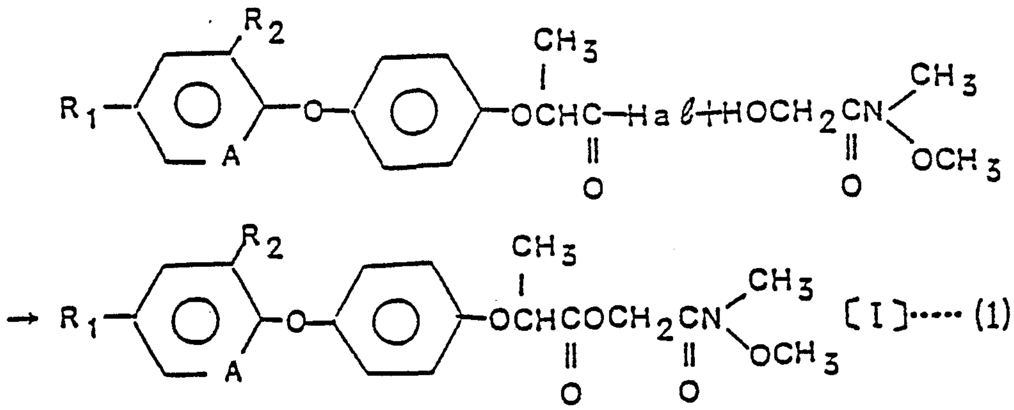

[I]····· (1)

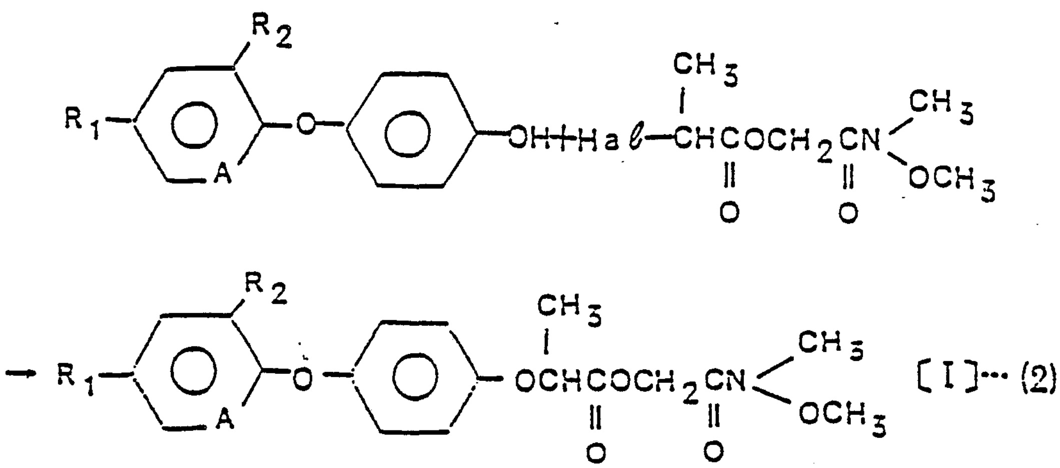

[I]··· (2)

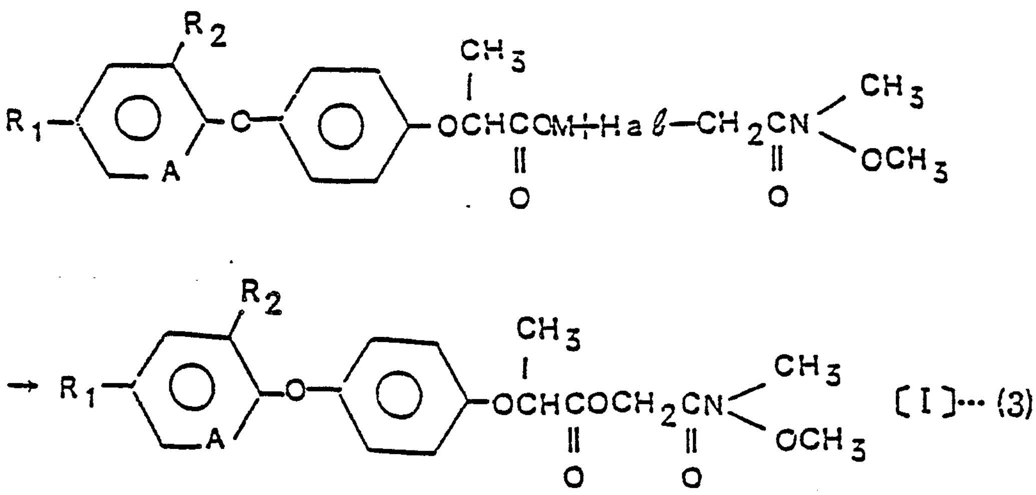

[I]··· (3)

In the above formulas, $R_1$ denotes a chlorine atom, bromine atom, trifluoromethyl group or nitro group; $R_2$ denotes a hydrogen atom or chlorine atom; A denotes a methine group or nitrogen atom; Hal denotes a halogen atom and M denotes a metal ion or ammonium ion. Among these manufacturing processes, the reactions represented by the processes (1) and (2) above will be explained in further detail in the following.

The compound of the present invention can be obtained by the above reactions either in the presence or in the absence of a reaction solvent. As reaction solvents, there can be used ketones such

as acetone and methyl ethyl ketone, aromatic hydrocarbons such as benzene, toluene and xylene, ethers such as ethyl ether, tetrahydrofuran and dioxane, halogenated hydrocarbons such as chlorobenzene, chloroform, carbon tetrachloride and dichloroethane, tertiary amines such as triethyl amine, pyridine and dimethylaniline, or polar solvents such as dimethylformamide, dimethylsulfoxide and hexamethyltriamide phosphate.

The reaction can be carried out in the presence of a base. Examples for suitable bases which can be used are tertiary amines such as triethyl amine, pyridine and dimethylaniline, alkali hydroxides such as sodium hydroxide and potassium hydroxide, alkaline earth metal hydroxides such as calcium hydroxide, alkaline carbonates such as sodium carbonate, potassium carbonate, sodium bicarbonate and potassium bicarbonate and metal hydrides such as sodium hydride.

The reaction normally takes place at about 0°C to 150°C, preferably between about 20°C and about 100°C. The time required for the reaction ranges from several minutes to about 48 hours.

In using the compound of the present invention as a herbicide, it can be used in any of the generally practiced forms such as an emulsion, a wettable powder, water soluble powder, oil soluble powder, dusting powder and as granules in combination with adjuvants. These formulations may be used either as they are or as dilutions (diluted to specified concentrations) with water, or on solid carriers.

The adjuvants include, for example, diluents, extending agents, surface active agents, stabilizers, fixing agents, spraying agents for aerosols and synergists. As solvents for diluents, there can be used water, organic solvents, hydrocarbons halogenated hydrocarbons, alcohols, ethers, alcohol ethers, ketones, esters, amides and sulfoxides. As the extending agents and the solid carriers, there can be used inorganic dust and granules such as slaked lime, magnesia lime, gypsum, calcium carbonate, silica, pearlite, pumice, diatomaceous earth, alumina, zeolite, clay minerals (talc, vermiculite, kaolinite), vegetable dust and granules (starch, farina, grape sugar) and synthetic resin dust and granules (phenol resin, urea resin, vinyl chloride resin). As the surface active agents, there can be used anionic surface active agents (alkyl sulfate esters, aryl sulfonic acids, succinic acid salts, polyethylene glycol alkyl aryl ether sulfate esters), cationic surface active agents (alkyl amines and polyoxyethylene alkyl amines), nonionic surface active agents (polyoxyethylene glycol ethers, polyoxyethylene glycol esters, polyhydric alcohol esters) and amphoteric surface active agents. In addition, there can be included stabilizers, fixing agents, efficacy extension agents, dispersion stabilizing agents and synergists.

In using the compound of the present invention as an active ingredient, the quantity thereof may be varied depending on the form of the formulation, the method, object and time of application and the condition of the weeds. In general applications, the compound of the present invention is used at the rate of 0.05 to 10 kg/ha, and preferably at 0.1 to 5 kg/ha.

The present invention will be further explained in the examples.

## EXAMPLE 1

2-[4-(4-trifluoromethylphenoxy)-phenoxy] propionic acid (N-methoxy-N-methyl-aminocarbonyl) methyl ester (Compound No. 1)

0.48 g of N-methoxy-N-methylhydroxy acetic acid amide was dissolved into 30 ml of dichloromethane, and 1.38 g of 2-[4-(4-trifluoro-methylphenoxy)-phenoxy] propionic acid chloride was dissolved into 10 ml of dichloromethane. Then the latter solution was dropped into the former solution at room temperature. After this solution had been stirred for 10 minutes, 0.39 g of triethyl amine was added, and the solution was stirred for 8 hours at room temperature. Water was added to the reaction mixture to separate dichloromethane, which was washed with water, and dried over anhydrus magnesium sulfate. The residue obtained by removing dichloromethane was refined by silica gel column chromatography [Benzene/ethyl acetate = 10/1 (v/v)]; and 1.11 g of the desired product was obtained. m.p. 78 -79°C.

## EXAMPLE 2

2-[4-(5-trifluoromethyl-2-pyridyloxy)-phenoxy] propionic acid (N-methoxy-N-methyl-aminocarbonyl) methyl ester (Compound No. 2)

2.6 g of 4-(5-trifluoromethyl-2-pyridyloxy)-phenol, 2.5 g of α-chloro-propionic acid (N-methoxy-N-methyl-aminocarbonyl) methyl ester and 1.65 g of potassium carbonate were added to 50 ml of methyl ethyl ketone, and the mixture was refluxed for eight hours while being stirred and heated. After cooling the reaction mixture, inorganic matters were separated by filtration and methyl ethyl ketone was distilled off. The residue was refined by silica gel column chromatography [benzene/ethyl acetate = 10/1 (v/v)], and 1.0 g of the desired product was obtained. m.p. 98 -101°C.

The following compounds were synthesized by methods similar to the above.

| Compound No. | Name of compound | Properties |
|---|---|---|
| 3. | 2-[4-(4-nitrophenoxy)-phenoxy] propionic acid (N-methoxy-N-methyl-aminocarbonyl) methyl ester | m.p. 96.5 - 97.5°C |
| 4. | 2-[4-(4-bromophenoxy)-phenoxy] propionic acid (N-methoxy-N-methyl-aminocarbonyl) methyl ester | $n_D^{25}$ 1.5361 |

| Compound No. | Name of compound | Properties |
|---|---|---|
| 5. | 2-[4-(3,5-dichloro-2-pyridyloxy)-phenoxy] propionic acid (N-methoxy-N-methyl-aminocarbonyl) methyl ester | $n_D^{25}$ 1.5510 |
| 6. | 2-[4-(2-chloro-4-trifluoro methylphenoxy)-phenoxy] propionic acid (N-methoxy-N- | |

| | | |
|---|---|---|
| | methyl-aminocarbonyl) methyl ester | $n_D^{25}$ 1.5234 |
| 7. | 2-[4-(2,4-dichlorophenoxy)-phenoxy] propionic acid (N-methoxy-N-methyl-amino carbonyl) methyl ester | m. p. 68 - 69°C |
| 8. | 2-[4-(3-chloro-5-trifluoro methyl-2-pyridyloxy)-phenoxy] propionic acid (N-methoxy-N-methyl-aminocarbonyl) methyl ester | $n_D^{25}$ 1.5269 |

EXAMPLE 3 Emulsion

20 parts (by weight; the same hereinafter) of compound No. 1 of the present invention, 60 parts of xylene and 20 parts of Sorpol®2806B - (Brandname of a product of Toho Kagaku Kogyo Co., Ltd.) were made into an emulsion by mixing them thoroughly by stirring.

EXAMPLE 4 Wettable powder

10 parts of white carbon, 65 parts of Zeeklite®-SP (Brandname of a product of Zeeklite Co., Ltd.), 5 parts of Sorpol®5039 (Brandname of a product of Toho Kagaku Kogyo Co., Ltd.) and 20 parts of the compound No. 3 of the present invention were mixed and crushed to obtain a wettable powder.

EXAMPLE 5 Soil treatment test

Soil was put in 1/5000 are Wagner's pots to create the upland condition, and certain quantities of the seeds of barnyard grass, Japanese radish and soybean were sowed. Then the soil in the pot was covered with a layer of soil about 1 cm in thickness containing the seeds of weeds of the Gramineae family such as the crab grass, barnyard grass, foxtail, etc. After 3 days from the sowing of the seeds, a liquid for spraying of a specified concentration was sprayed evenly on the soil. After 22 days from the treatment of the soil, the conditions of growth of the vegetables and the weeds were observed. The result are shown in Table 1. The values representing the degree of growth control (of the weeds) were classified into 5 grades (5 to 1) (5 denotes complete destruction, and 1 no effect).

Table 1

| Compound No. | Amount of active ingredient kg/ha | Degree of growth control | | | |
|---|---|---|---|---|---|
| | | Japanese radish | Soybean | Barnyard grass | Weed of Gramineae family |
| 1 | 1 | 1 | 1 | 5 | 5 |
| | 0.5 | 1 | 1 | 5 | 5 |
| 2 | 1 | 1 | 1 | 5 | 5 |
| | 0.5 | 1 | 1 | 5 | 5 |
| 5 | 1 | 1 | 1 | 5 | 5 |
| | 0.5 | 1 | 1 | 5 | 5 |
| 7 | 1 | 1 | 1 | 5 | 5 |
| | 0.5 | 1 | 1 | 4 - 5 | 4 |
| Comparative compound (A) | 1 | 1 | 1 | 5 | 5 |
| | 0.5 | 1 | 1 | 5 | 4 |
| Comparative compound (B) | 1 | 1 | 1 | 5 | 5 |
| | 0.5 | 1 | 1 | 5 | 4 |
| Comparative compound (C) | 1 | 1 | 1 | 5 | 4 |
| | 0.5 | 1 | 1 | 4 | 3 |
| Untreated section | - | 1 | 1 | 1 | 1 |

Comparative compound (A)

2-[4-(4-trifluoromethylphenoxy)-phenoxy] propionic acid ethyl ester.

Comparative compound (B)

2-[4-(5-trifluoromethyl-2-pyridyloxy)-phenoxy] propionic acid butyl ester.

Comparative compound (C)

2-[4-(2,4-dichlorophenoxy)-phenoxy] propionic acid methyl ester.

EXAMPLE 6 Leaves and caules treatment test

When the weeds which had been grown by the same method as that of EXAMPLE 5 had entered 3 -3.5 leaf period, the chemical of the specified concentration was sprayed evenly over the leaves and the caules. After 5 days and 10 days from the treatment, the condition of the growth of the weeds was observed, and obtained the result shown in Table 2. The values of the degree of the growth control of the weeds are similar to those in the case of EXAMPLE 5.

Table 2

| Compound No. | Concentration of active ingredient ppm | 5 days after treatment | | | | 10 days after treatment | | | |
|---|---|---|---|---|---|---|---|---|---|
| | | Barnyard grass | Oat | Foxtail | Crab grass | Barnyard grass | Oat | Foxtail | Crab grass |
| 1 | 500 | 5 | 5 | 5 | 5 | – | – | – | – |
| | 250 | 5 | 5 | 5 | 5 | – | – | – | – |
| 2 | 500 | 5 | 5 | 5 | 5 | – | – | – | – |
| | 250 | 5 | 5 | 5 | 5 | – | – | – | – |
| 5 | 500 | 5 | 5 | 5 | 5 | – | – | – | – |
| | 250 | 5 | 5 | 5 | 5 | – | – | – | – |
| 7 | 500 | 5 | 5 | 5 | 5 | – | – | – | – |
| | 250 | 4 | 2 | 4 | 5 | – | – | – | – |
| Comparative compound (A) | 500 | 1 | 1 | 1 | 1 | 5 | 5 | 5 | 5 |
| | 250 | 1 | 1 | 1 | 1 | 5 | 4 | 5 | 5 |
| Comparative compound (B) | 500 | 1 | 1 | 1 | 1 | 5 | 4 | 5 | 5 |
| | 250 | 1 | 1 | 1 | 1 | 5 | 3 | 5 | 5 |
| Comparative compound (C) | 500 | 1 | 1 | 1 | 1 | 5 | 3 | 5 | 5 |
| | 250 | 1 | 1 | 1 | 1 | 4 | 1 | 3 | 4 |
| No treatment | – | 1 | 1 | 1 | 1 | 1 | 1 | 1 | 1 |

Comparative compounds (A), (B) and (C) are the same as those in the case of EXAMPLE 5.

The compounds of the present invention have a satisfactory selectivity concerning vegetables such as beans, cotton, carrots, potatoes, beet, cabbage, mustard, peanuts, Japanese radish, tobacco, tomatoes,cucumbers, etc., and when these compounds (having the usual concentrations for the usual use) are applied for the pre-emergence treatment and post-emergence treatment of weeds, especially of the weeds of the Gramineae family, such as barnyard grass, crab grass, goose grass, foxtail, wild oats, water foxtail, yellow foxtail, quack grass, Agropyron tsukushiens, Bermuda grass, Johnson grass, etc., they display an excellent selective herbicidal effect.

Also, when the compounds of the present invention are used for post-emergence treatment, the period of time required for the development of the activity of the compound is 4 to 5 days, which is much shorter than that of similar conventional compounds, and this is one of the outstanding features of each of the compounds of the present invention.

## Claims

1. A derivative of propionic acid represented by the following general structural formula [1]:

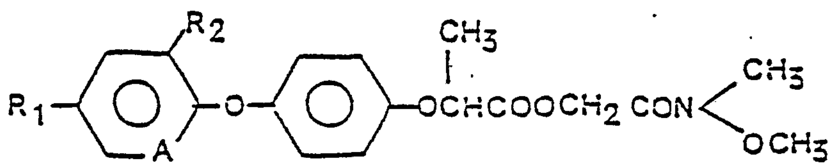

[I]

wherein $R_1$ denotes a chlorine atom, bromine atom, trifluoromethyl group or nitro group; $R_2$ denotes a hydrogen atom or chlorine atom; and A denotes a methine group or nitrogen atom.

2. The compound according to claim 1 which is 2-[4-(4-trifluoromethylphenoxy)-phenoxy] propionic acid (N-methoxy-N-methyl-aminocarbonyl) methyl ester.

3. The compound according to claim 1 which is 2-[4-(5-trifluoromethyl-2-pyridyloxy)-phenoxy] propionic acid (N-methoxy-N-methyl-aminocarbonyl) methyl ester.

4. The compound according to claim 1 which is 2-[4-(4-nitrophenoxy)-phenoxy] propionic acid (N-methoxy-N-methyl-aminocarbonyl) methyl ester.

5. The compound according to claim 1 which is 2-[4-(4-bromophenoxy)-phenoxy] propionic acid (N-methoxy-N-methyl-aminocarbonyl) methyl ester.

6. The compound according to claim 1 which is 2-[4-3,5-dichloro-2-pyridyloxy)-phenoxy] propionic acid (N-methoxy-N-methyl-aminocarbonyl) methyl ester.

7. The compound according to claim 1 which is 2-[4-2-chloro-4-trifluoromethylphenoxy)-phenoxy] propionic acid (N-methoxy-N-methyl-aminocarbonyl) methyl ester.

8. The compound according to claim 1 which is 2-[4-(2,4-dichlorophenoxy)-phenoxy] propionic acid (N-methoxy-N-methyl-aminocarbonyl) methyl ester.

9. The compound according to claim 1 which is 2-[4-(3-chloro-5-trifluoromethyl-2-pyridyloxy)-phenoxy] propionic acid (N-methoxy-N-methyl-aminocarbonyl) methyl ester.

10. A selective herbicidal composition containing an herbicidally effective amount of the derivative of the propionic acid [1] according to any of claims 1, 2, 3, 4, 5, 6, 7, 8 and 9.

11. A method for selectively controlling weeds of the Gramineae family coexisting with cultivated plants by using for pre-emergence treatment or post-emergence treatment a herbicidally-effective amount of the derivative of a propionic acid [1] according to any of claims 1, 2, 3, 4, 5, 6, 7, 8 and 9.